Europäisches Patentamt

European Patent Office

Office européen des brevets

(19)

(11) **EP 1 672 076 A1**

(12)

# EUROPEAN PATENT APPLICATION
published in accordance with Art. 158(3) EPC

(43) Date of publication:
   **21.06.2006 Bulletin 2006/25**

(51) Int Cl.:
   ***C12N 15/867*** (2000.01)     ***C12N 5/10*** (1990.01)
   ***A01K 67/027*** (1990.01)

(21) Application number: **04772344.0**

(22) Date of filing: **27.08.2004**

(86) International application number:
   **PCT/JP2004/012388**

(87) International publication number:
   **WO 2005/021769 (10.03.2005 Gazette 2005/10)**

(84) Designated Contracting States:
   **AT BE BG CH CY CZ DE DK EE ES FI FR GB GR
   HU IE IT LI LU MC NL PL PT RO SE SI SK TR**

(30) Priority: **29.08.2003 JP 2003306573**

(71) Applicants:
   • **KANEKA CORPORATION**
     **Osaka-shi, Osaka 530-8288 (JP)**
   • **Nagoya Industrial Science Research Institute**
     **Nagoya-shi, Aichi 460-0008 (JP)**

(72) Inventors:
   • **IIJIMA, Shinji**
     **Nagoya-shi,**
     **Aichi 4680022 (JP)**

   • **KAMIHIRA, Masamichi,**
     **603, Takaramanshonshirokicho**
     **Nagoya-shi,**
     **Aichi 4640846 (JP)**
   • **NISHIJIMA, Kenichi,**
     **302, Kuzuokamanshon**
     **Nagoya-shi,**
     **Aichi 4640804 (JP)**

(74) Representative: **HOFFMANN EITLE**
   **Patent- und Rechtsanwälte**
   **Arabellastraße 4**
   **81925 München (DE)**

(54) **METHOD OF CONSTRUCTING TRANSGENIC BIRD USING LENTIVIRUS VECTOR AND TRANSGENIC BIRD OBTAINED THEREBY**

(57)    The present invention provides a lentivirus vector which comprises a coat protein containing VSV-G; a recombinant bird host which is constructed by infecting a bird host with the lentivirus vector; a method of constructing the same; a transgenic chimeric bird which is constructed by infection with the lentivirus vector and offspring thereof. The present invention also provides a method of constructing a G0 transgenic chimeric bird and offspring thereof which comprises infecting fertilized bird egg early embryos with a lentivirus vector comprising a coat protein containing VSV-G, and incubating the embryos; and a G0 transgenic chimeric bird and off spring thereof which is constructed by the method mentioned above.

   The present invention provides a transgenic bird which is constructed by infecting a bird early embryos with a lentivirus vector comprising a coat protein pseudotyped with VSV-G.

Fig. 1

EP 1 672 076 A1

**Description**

TECHNICAL FIELD

This invention relates to a method of introducing an exogenous gene into a cultured bird cell using a lentivirus vector. The invention also relates to a method of constructing transgenic chimeric birds by introducing an exogenous gene into fertilized egg early embryos using a lentivirus vector and to G0 transgenic chimeric birds obtained by said method.

BACKGROUND ART

In recent years, a number of antibody drugs have been put on the market. However, they are currently produced only by means of animal cell reactors and therefore are expensive. This is a dominant cause of restricted spread thereof. The transgenic animal factory technology has been attracted attention as means for cheaply producing proteins having a complicated structure and requiring a sugar chain for their stability and activity manifestation, for example monoclonal antibodies to be used as drugs.

The attempts to enable mammals such as cows, goats and sheep to produce physiologically active substances cheaply in the milk will soon be put to practical use. In the case of these animals, however, it is a drawback that the period between the construction of transgenics to the initial production of the desired products is as long as 1 to 2 years.

On the other hand, transgenic birds the sexual maturation period of which is short and which are to produce physiologically active substances in eggs thereof are promising animal factories since domestic fouls are efficient protein-producing animals. In constructing stable transgenics by introducing an exogenous gene into bird somatic cells, however, particular difficulties are encountered. Hence, transgenic birds have not yet put to practical use (cf. WO 03/014344 A2, Viragen Incorporated (2001)).

One of the reasons why the construction of transgenic birds is difficult is that it is very difficult to obtain fertilized eggs thereof at the one-cell stage and artificially cultivate them. Perry operatively obtained precleavage chicken cells and established a system for extracorporeally cultivating them (cf. Perry, M. M. (1988), Nature, 331). However, it is still impossible to construct transgenics by nucleus transplantation using this technology, since the bird cell pronucleus is covered with yolk and therefore is indistinguishable.

Therefore, the exogenous gene transfer into fertilized bird eggs is restricted to gene injection into cytoplasm by lipofection or electroporation, for example. It is impossible to intentionally integrate a transgene into a chromosome.

As a result of intensive investigations made by the present inventors, they found out a method of constructing transgenic birds by using a replication-defective retrovirus vector, which is safe and is also applied in gene therapy, for efficient introduction of a desired gene (cf. Japanese Kokai Publication 2002-176880). This has made it possible to construct transgenic birds carrying a plurality of copies of a transgene in a safe and efficient manner. It was also found that when this technique is used, the transgene can be transmitted to the next generation with high.efficiency, bringing the utilization of transgenic birds as substance production systems close to practical use.

The inventors also found out a method of causing protein expression based on the novel finding that the inactivation of a gene transferred with a retrovirus depends on the time of transfer.

The method of gene transfer using a retrovirus vector is currently the only method of constructing transgenic birds by integrating an exogenous gene into a chromosomal DNA with high transfer efficiency. However, it is a problem that the exogenous gene transferred with a retrovirus vector is inactivated by methylation (the phenomenon called silencing), with the result that the desired protein cannot be expressed.

The silencing phenomenon is considered to be a kind of protective system of organisms but presents an obstacle in constructing transgenics as animal factories.

The lentivirus vector developed by Verma et al. is known to be capable of allowing gene transfer even into cells that have differentiated and stopped proliferating, for example nervous system cells without accompaniment of inactivation of the transgene, and it is tolerant of silencing (cf. Verma, I. M. et al. (2000) Nat. Rev. Genet. 1, 91).

Pfeifer et al. succeeded in constructing transgenic mice using such lentivirus vector and reported about the exogenous protein expression in somatic cells thereof (cf. Pfeifer, A. et al. (2002) Proc. Natl. Acad. Sci. USA, 99, 2140).

An attempt to apply this lentivirus vector in constructing transgenic birds has been reported. Generally, however, bird cells are not infected with lentiviruses.

The inventors have made attempts to develop an effective method of gene transfer into birds based on the finding that silencing hardly occurs in certain retrovirus species, side by side investigations concerning the practical use of transgenic birds through elucidation of the mechanisms of transgene inactivation, as mentioned above. The application of vectors derived from lentiviruses belonging to the family of retroviruses is one of such attempts.

Since, however, bird cells are not infected with lentiviruses as such, it is necessary to develop lentivirus vectors capable of infecting bird cells so that lentiviruses may be applied in constructing transgenic birds.

Lentivirus vectors are a kind of retrovirus vectors, as mentioned above, but they have different properties as compared with common retrovirus vectors, for example vectors derived from Moloney murine leukemia virus (MoMLV).

Generally, retrovirus vectors infect only cells in the growth phase, so that they cannot serve in gene transfer into cells in the quiescent state after differentiation. However, lentivirus-derived novel vectors can infect not only proliferating cells but also quiescent cells, for example nerve cells and, therefore, have a wide range of application. It is also known that the transgene inactivation (silencing), which is a problem with ordinary retroviral vectors, does not occur in them.

There is no report about the infection of bird cells with a lentivirus and, therefore, for applying lentivirus vectors in constructing transgenic birds, it is necessary to contrive to infect bird cells with the vector.

SUMMARY OF THE INVENTION

The present inventors made various investigations and found that a lentivirus vector comprising a coat protein pseudotyped with VSV-G can allow exogenous gene transfer into cultured chicken cells. Such finding has now led to completion of the present invention.

Thus, the invention provides, in a first aspect thereof,
a method of constructing a transgenic bird and offspring thereof
which comprises infecting fertilized bird egg early embryos with a VSV-G pseudotyped lentivirus vector; and,
in a second aspect thereof, the invention provides
a lentivirus vector, a recombinant bird host and a transgenic bird constructed by said method.

More specifically, the invention provides
a lentivirus vector
which comprises a coat protein containing VSV-G;
a recombinant bird host
which is constructed by infecting a bird host with said lentivirus vector;
a method of constructing said recombinant bird host;
a transgenic chimeric bird
which is constructed by infection with said lentivirus vector, and
offspring thereof;
a method of constructing a G0 transgenic chimeric bird and offspring thereof
which comprises infecting fertilized bird egg early embryos with a lentivirus vector comprising a coat protein containing VSV-G, and incubating the embryos; and
a G0 transgenic chimeric bird and offspring thereof
which is constructed by the method mentioned above.

Pfeifer et al. referred to above infected mouse fertilized eggs with a lentivirus by treating the fertilized eggs with acidic Tyrode' s solution to thereby removing the clear zone. The VSV-G pseudotyped lentivuses created by the present inventors can infect untreated bird cells. The infectious lentivirus vectors facilitating the pretreatment of cells constitute an aspect of the present invention.

One of the advantages of the application of lentivirus vectors in constructing transgenic birds is that it is highly possible to produce full-body offspring (G1) by gene transfer into the germline. Another advantage is that the transgene-derived protein production increases as a result of the avoidance of silencing.

Thus, the invention discloses a method of constructing transgenic birds favorable for protein production. It also discloses transgenic birds constructible by such method.

The transgenic birds constructed by the above method can be utilizing in protein production and, further, in bird breed improvement.

DETAILED DESCRIPTION OF THE INVENTION

In the following, the invention is described in detail.

As the lentivirus vector to be used in accordance with the invention, there may be mentioned HIV (Human immunodeficiency virus)-derived and BIV (Bovine immunodeficiency virus)-derived lentivirus vectors, and the like. Preferred as the lentivirus vector to be used in the practice of the invention are HIV-derived ones. More preferred are HIV-1-derived ones.

From the safety viewpoint, it is desirable that the virus to be used as the gene transfer vector be replication-defective as a result of elimination of the regulator genes necessary for replication and, at the same time, be such that the expression of the virus is effected by later adding plasmids carrying the genes necessary for virus particle replication as divided into gene segments.

In accordance with the invention, a virus vector comprising a coat protein artificially pseudotyped with VSV-G (derived from vesicular stomatitis virus) is used to enable the infection of host birds with the virus vector. The receptor of VSV-G is a phospholipid, which is a membrane constituent, and this commonly occurs in bird cell membranes as well and thus makes lentiviruses, which generally cannot infect bird hosts, capable of infecting them.

As the bird hosts, there may be mentioned fertilized bird egg early embryos and bird host cells, and the like.

The pseudotyped virus vector prepared by utilizing packaging cells, a helper virus or the like is introduced into fertilized bird egg early embryos and also introduced into bird host cells via intravascular and intracardiac routes preferably by the conventional microinjection technique (Bosselman, R. A. et al. (1998) Science 243, 533). Conceivable as other methods of gene transfer are lipofection and electroporation, and the like.

As bird host cells, there may be mentioned, for example, fibroblasts, pluripotent stem cells, primordial germ cells and the like. A recombinant bird host which is constructed by infecting a bird host with said lentivirus vector, and a method of constructing said recombinant bird host also constitute aspects of the present invention.

The lentivirus vector to be used in the practice of the invention is preferably a heterologous gene-containing one.

The heterologous gene to be introduced into birds using the lentivirus vector in accordance with the invention is not particularly restricted but is constituted of a marker gene or a structural gene for the expression of a desired protein, a promoter gene controlling the expression of those genes, a secretory signal gene and the like. The heterologous gene preferably contains a structure gene and, more preferably, further contains a marker gene.

As the marker gene, there may be mentioned the neomycin resistance gene, LacZ ($\beta$-galactosidase gene), and the gene coding for a fluorescent protein such as GFP (green fluorescent protein) . The lentivirus vector to be used in the practice of the invention is preferably one with the GFP gene and/or LacZ inserted therein.

The structural gene for the expression of a desired protein is not particularly restricted but includes, for example, genes coding for antibodies useful in the genetic industry, such as human monoclonal antibodies, enzymes or the like, or genes coding for single-strand antibodies. Furthermore, genes coding for other useful physiologically active substances can also be used. Particularly preferred are structural genes coding for exogenous antibodies, for example genes coding for antibodies having a constant region of the human IgG class and genes coding for antibodies having a constant region of the human IgG1, quail IgG2, chicken IgG2 or mouse IgG2 subclass, since such antibodies are abundantly accumulated in eggs.

As preferred structural genes for the expression of the desired proteins mentioned above, there may be mentioned structural genes coding for chimera antibodies. The term "chimera antibody" refers to an antibody comprising two or more different inherited characters.

The antibodies for medical use so far produced by mouse hybridomas are of the mouse origin and, therefore, it is a problem that when administered into the human body, they cause rejection by the immune system. As the chimera antibodies, there may be mentioned, for example, those chimera antibodies in which the above drawback has been remedies and which cause no rejection, for example anti-human CD2 antibodies, anti-CD20 receptor antibodies, anti-TNF antibodies and the like. Some of them are already on the market as medicines.

By using such human monoclonal antibody genes or chimera antibody genes as the genes to be introduced into birds to give the G0 transgenic chimeric birds according to the invention, it becomes possible to produce antibodies for medical use, which have so far been difficult to produce, in large amounts and at low cost.

As the promoter gene, there may be mentioned constitutive promoters. When an antibody gene is controlled under a constitutive promoter, the expression of the antibody gene is favorably stable. As a more preferred constitutive promoter, there may be mentioned the chicken $\beta$ actin promoter.

The G0 transgenic chimeric birds and offspring thereof as constructed in accordance with the invention by introduction of an exogenous antibody gene by means of a lentivirus vector can produce the transgene-derived protein in blood, egg white or yolk. The proteins thus produced undergo sugar chain modification in bird cells and this is advantageous from the activity and stability viewpoint.

The birds to be used in the practice of the invention are not particularly restricted but there may be mentioned, for example, chickens, turkeys, ducks, ostriches, quails and other domestic fowls domesticated for eating as meats or collecting eggs as well as pet birds. Among them, chickens and quails are readily available and preferred because of their being prolific as egg-laying species. More preferred are chickens.

The method of constructing G0 transgenic chimeric birds and offspring thereof according to the invention is now described.

The method of constructing G0 transgenic chimeric birds and offspring thereof according to the invention comprises infecting fertilized bird egg early embryos with a lentivirus vector comprising a coat protein containing VSV-G, and incubating the embryos.

As an example of such method for construction, there may be mentioned the method comprising infecting early embryos at the blastodermic stage (stage X) just after laying of fertilized bird eggs with a replication-defective lentivirus vector, and incubating the embryos. Also employable is the method which comprises incubating fertilized bird eggs, infecting early embryos thereof at the state succeeding the blastodermic stage just after being laid with a replication-defective lentivirus vector, and incubating the embryos. As another example of the method of the invention, there may be mentioned the method comprising incubating fertilized bird eggs, infecting early embryos thereof after at least 24 hours from the start of incubation with a replication-defective lentivirus vector, and incubating the embryos.

More preferred is the method comprising microinjecting a replication-defective lentivirus vector into the heart or blood vessel formed in the early embryos.

In carrying out the method of constructing G0 transgenic chimeric birds and offspring thereof according to the invention, a replication-defective lentivirus vector is preferably microinjected into fertilized eggs just after being laid (at stage X).

As for the early development of a fertilized egg after being laid in a chicken, for example, the egg fertilized in the oviduct begins cleavage in about 1.5 hours after fertilization. The egg that has begun discoidal cleavage with the cytoplasm still undivided cleaves over a period of 1 day until discharge from the body, to form an embryo, called blastoderm, consisting of about 60 thousand cells (blastodermic stage). This blastoderm is observed as a white ring, 3 to 4 mm in diameter, in the central part of the yolk. This embryo cleaves into an upper and a lower layer to form a blastocoel. The egg is laid when the hypoblast is formed, the primitive streak is then formed, and the blastoderm takes a three-layer structure consisting of an upper layer, a middle layer and a lower layer. Three germ layers are thus formed. Then, after embryogenesis and growth, hatching occurs on the 22nd day after ovulation. The blastodermic stage is also called stage X, and reproductive cells are formed from a part of the cells at this stage. Therefore, fertilized eggs at this stage are conventionally used as targets of gene transfer.

In the practice of the invention, the stage for introducing a heterologous gene into embryos is not particularly restricted but may be before stage X, at stage X or after stage X. From the viewpoint that silencing hardly occurs, the heterologous gene is preferably introduced into embryos after stage X, more preferably after the lapse of 48 hours from the start of incubation, most preferably at 55 hours from the start of incubation.

In the practice of the invention, the time when fertilized eggs at the blastodermic stage just after being laid were placed under environmental conditions suited for hatching (in the case of chickens, for example, a temperature of 37.7 to 37.8°C and a humidity of about 50 to 70%) was taken as time 0 (zero), and microinjection was carried out at timed intervals. The formation of a blood circulatory system on the yolk was observed and the pulsation of an organ to differentiate into the heart could be observed after 36 hours from the start of incubation in quails, or after about 50 hours from the start of incubation in chickens.

For the hatching of fertilized eggs after the above gene transfer, the method using artificial eggshells as developed by the present inventors (Kamihira, M. et al. (1998) Develop. Growth Differ., 40, 449), for example, can be applied.

As the lentivirus vector, the transgene and the bird to be subjected to gene transfer, which are to be used in carrying out the method for construction of the invention, there may be mentioned the same ones as described hereinabove.

In the method for construction of the invention, the "gene not derived from lentivirus" includes marker genes, structural genes, promoter genes and secretory signal genes.

In carrying out the method for construction of the invention, the time for lentivirus vector infection is preferably at the blastodermic stage. The time for lentivirus vector infection is also preferably at a period succeeding the blastodermic stage and, in that case, the site of lentivirus vector infection is preferably in the early heart or early blood vessel.

In carrying out the method for construction of the invention, the method of lentivirus vector infection is preferably the microinjection method.

In carrying out the method for construction of the invention, injection, preferably microinjection, of a lentivirus vector having a titer of not lower than $1 \times 10^5$ cfu/mL, more preferably not lower than $1 \times 10^6$ cfu/mL, is preferred, since, then, the gene transfer can be carried out efficiently.

In the practice of the invention, the virus titer is measured by the X-Gal staining method or fluorescent protein detection method.

The birds constructed by the gene transfer into fertilized eggs thereof by the method for construction of the invention grow as transgenic birds carrying the transgene in a mosaic manner in their somatic cells. These first generation transgenic birds are referred to as G0 transgenic chimeric birds.

The G0 transgenic chimeric birds and offspring thereof obtained by such method for construction of the invention also constitute an aspect of the present invention.

The offspring of the G0 transgenic chimeric birds of the invention can be produced by any of the breeding technologies known in the art. The related technologies are well known in the relevant field of art and include, but are not limited to, hybridization, inbreeding, backcross, multiple cross, interspecific hybridization and the like.

More specifically, each individual of the second generation and third generation born as a result of mating of a G0 transgenic chimeric bird with a non-transgenic bird or mating of a G0 transgenic chimeric bird with a G0 transgenic chimeric bird, when it has developed from a reproductive cell containing the transgene on a chromosome thereof, grows as an individual containing the transgene in the somatic cells of the whole body. The offspring inheriting the transgene from G0 transgenic chimeric bird individuals are referred to successively as G1, G2, G3 ... transgenic birds.

By mating a G0 transgenic chimeric bird according to the invention with a non-transgenic bird of the same species or with a mating type G0 transgenic chimeric bird, it becomes possible to transmit the transgene to their offspring and at the same time to construct fully transgenic birds carrying the transgene in the somatic cells of the whole body. As compared with G0 transgenic chimeric birds, such fully transgenic birds can be expected to produce the transgene-derived recombinant protein in increased amounts, since the proportion of the trans gene-containing somatic cells is higher. Furthermore, by establishing a line of transgenic birds capable of stably transmitting the transgene, it becomes possible to stabilize the quality as a protein production system.

In accordance with the invention, G0 transgenic chimeric birds can be constructed by hatching fertilized bird egg early embryos infected with a lentivirus vector comprising a coat protein pseudotyped with VSV-G. The method of constructing G0 transgenic chimeric birds and offspring thereof according to the invention makes it possible to introduce the gene coding for a physiologically active protein, for example an antibody for medical use, to construct birds capable of efficiently expressing the antibody in the blood or eggs. Furthermore, the method of producing proteins using the G0 transgenic chimeric birds and offspring thereof according to the invention enables efficient antibody production by constructing G0 transgenic chimeric birds and offspring thereof capable of producing an antibody for medical use, for example, and recovering and purifying the antibody from the serum and/or eggs of the birds.

BRIEF DESCRIPTION OF THE DRAWINGS

Fig. 1 shows the structure of the vector construct pLenti6/CMV-lacZ for β-galactosidase expression prepared in Example 1 to be described below. Blasticidin denotes the blasticidin resistance gene, and Ampicillin denotes the ampicillin resistance gene. Ψ denotes the packaging signal sequence. CMVPro denotes the CMV promoter gene. LacZ denotes the gene for β-galactosidase expression. 5'LTR and 3'LTRΔU3 respectively denote the CMV long terminal repeat sequences.

Fig. 2 shows the differences in β-galactosidase activity among cell species. The abscissa denotes the amount added of the virus solution having a titer of $(1.7 \pm 0.3) \times 10^6$ cfu/mL. The ordinate denotes the β-galactosidase activity expressed in terms of units. Mock denotes a control.

Fig. 3 shows the structure of the vector construct pLenti/cDf-ΔAsPE-W for anti-prion scFvFc and green fluorescent protein (GFP) expression. bla denotes the blasticidin resistance gene. Ψ denotes the packaging signal sequence.

pact denotes the chicken β-actin promoter gene. scFv-Fc denotes the single strand antibody structural gene. EGFP denotes the green fluorescent protein (GFP) structural gene.

BEST MODES FOR CARRYING OUT THE INVENTION

The following examples illustrate the present invention in detail. These examples are, however, by no means limitative of the scope of the invention.

(Example 1) Preparation of vector construct pLenti6/CMV-lacZ for β-galactosidase expression

The pLenti6/CMV-lacZ vector construct was constructed in the following manner.

First, pLenti6/TOPO-GFP was constructed in the following manner. Two primers for amplifying GFP, namely 5'-CACCATGAGCAAGGGC-3' (SEQ ID NO:1) and 5'-TCACTTGTACAGCTCGTCCA-3' (SEQ ID NO:2), were chemically synthesized. Using pGreen Lantern (product of Gibco) as a template, PCR was carried out to amplify a GFP fragment. This amplification product solution was subcloned into the pLenti6/V5-D-TOPO vector according to the manual attached to pLenti6/V5 Directional TOPO Cloning Kit (product of Invitrogen Corporation), whereby pLenti6/TOPO-GFP was constructed.

Furthermore, pLNΔAβ was constructed in the following manner.

1. A Rous sarcoma virus (RSV) promoter fragment was cleaved from the plasmidpLXRN (product of Clontech Laboratories, Inc.) with the restriction enzymes XhoI and HindIII and inserted into the plasmid pBluescriptIISK (+) (product of Stratagene) at the XhoI-HindIII site. A plasmid, pBlue/RSV, was thus constructed.

2. A β-galactosidase (β-Gal) gene fragment was cleaved from the plasmid pCMVβ (product of Clontech Laboratories, Inc.) with the restriction enzyme NotI and inserted into the plasmid pZeoSV2 (+) (product of Invitrogen Corporation) at the NotI site. The plasmid having a structure resulting from insertion of the β-Gal gene in the same direction as the T7 promoter was designated as pZeo/lacZ.

3. A RSV promoter fragment was cleaved from pBlue/RSV with the restriction enzymes XhoI and PstI. A β-Gal gene fragment was cleaved from pZeo/lacZ with the restriction enzymes PstI and XhoI. The above two fragments cleaved were joined to a vector fragment prepared by treatment of the plasmid pLNHX (product of Clontech Laboratories, Inc.) with the restriction enzyme XhoI, whereby a plasmid, pLNRβ, was constructed.

4. A fragment containing the series of the Moloney murine sarcoma virus (MoMuSV) 5'-long terminal repeat (LTR), virus packaging signal and neomycin resistance (Neo[r]) gene was cleaved from pLNHX with the restriction enzymes SacII and XhoI and joined to a vector fragment prepared by treatment of pLXRN with the restriction enzymes SacII and XhoI. A plasmid, pLXL, was thus constructed.

5. A β-Gal gene fragment was cleaved from pZeo/lacZ with the restriction enzymes HindIII and XhoI and joined to a vector fragment prepared by treatment of pLXL with the restriction enzymes HindIII and XhoI. A plasmid, pLZL, was thus constructed.

6. A 5' region fragment of the hybrid promoter (Miw promoter) derived from the RSV promoter and chicken β-actin (Act) promoter was amplified from the plasmid pMiwZ (Suemori et al., 1990, Cell Diff. Dev. 29:181-185) by PCR (94°C/15 seconds → 55°C/30 seconds → 72°C/1 minute and 30 seconds: 35 cycles; KOD-Plus-DNA polymerase (product of TOYOBO CO., LTD.)) using, as primers, two chemically synthesized oligonucleotides, 5'-cggtctagaggaattcagtggttcg-3' (SEQ ID NO:3) and

5'-ccaggatccgacgttgtaaaacgacg-3' (SEQ ID NO:4; the underlined portion being the BamHI restriction site), followed by cleavage thereof with the restriction enzymes BamHI and MunI. The cleaved fragment was inserted into the plasmid pGREEN LANTERN-1 (product of Gibco BRL) at the BamHI-MunI site. A plasmid, pGmiw5', was thus constructed.

7. AMiw promoter 5' side central region fragment was cleaved from pMiwZ with the restriction enzymes MunI and ClaI. By inserting that fragment into pGmiw5' at the MunI-ClaI site, a plasmid, pGmiw5'-2, was constructed.

8. A fragment containing the Miw promoter 5' region and 5' side central region was cleaved from pGmiw5'-2 with the restriction enzymes BamHI and EcoRI. By inserting that fragment into pBluescriptIISK(+) at the BamHI-EcoRI site, a plasmid, pBlue/Miw5', was constructed.

9. A Miw promoter 3' region fragment was amplified from pMiwZ by PCR (98°C/15 seconds → 60°C/30 seconds → 72°C/30 seconds: 35 cycles) using, as primers, two chemically synthesized oligonucleotides, 5'-ccaaagcttgccgcagccattgcctttt-3' (SEQ ID NO:5; the underlined being the HindIII restriction site) and 5'-atacctaggggctggctgcggaggaac-3' (SEQ ID NO:6; the underlined portion being the BlnI restriction site), followed by cleavage thereof with the restriction enzymes HindIII and BlnI. By joining the cleaved fragment to a vector fragment prepared by treatment of pLXL with the restriction enzymes HindIII and BlnI, a plasmid, pLMiw3', was constructed.

10. A Miw promoter 3' side central region fragment was cleaved from pMiwZ with the restriction enzymes EcoRI and MboII. Furthermore, a Miw promoter 3' region fragment was cleaved from pLMiw3' with the restriction enzymes MboII and KpnI. The two fragments cleaved were inserted into pBlue/Miw5' at the EcoRI-KpnI site. A plasmid, pBlue/Miw, was thus constructed.

11. A fragment containing the full-length Miw promoter was cleaved from pBlue/Miw with the restriction enzymes BamHI and BlnI. This fragment was joined to a vector fragment prepared by treatment of pLXL with the restriction enzymes BamHI and BlnI. A plasmid, pLML, was thus constructed.

12. An Act promoter fragment was cleaved from pLML with the restriction enzymes SmaI and XbaI. The fragment was inserted into pBluescriptIISK(+) at the EcoRV-XbaI site. A plasmid, pBlue/Act, was thus constructed.

13. A Miw promoter fragment was cleaved from pLML with the restriction enzymes HindIII and BglII. This fragment was joined to a vector fragment prepared by treatment of pLZL with the restriction enzymes HindIII and BamHI. A plasmid, pLMβL, was thus constructed.

14. An Act promoter fragment was cleaved from pBlue/Act with the restriction enzymes SalI and BlnI. A β-Gal gene fragment was cleaved from pLMβL with the restriction enzymes BlnI and BglII. The above-mentioned two fragments cleaved were joined to a vector fragment prepared by treatment of pLNRβ with the restriction enzymes XhoI and BglII. A plasmid, pLNAβ, was thus constructed.

15. An intron-deficient actin (ΔAct) promoter fragment was amplified from pMiwZ by PCR (98°C/15 seconds → 60°C/30 seconds → 68°C/2 minutes: 30 cycles) using, as primers, two chemically synthesized oligonucleotides, 5'-tttagctagctgcagctcagtgcatgcac-3' (SEQ ID NO:7; the underlined portion being the NheI restriction site) and 5'-ataatctagaaacgcagcgactcccgc-3' (SEQ ID NO:8; the underlined portion being the XbaI restriction site), followed by cleavage of a fragment containing a part of the ΔAct promoter with the restriction enzymes XhoI (the XhoI restriction enzyme site occurring in the fragment amplified) and XbaI . A fragment containing the remaining portion of the ΔAct promoter and the β-Gal gene was cleaved from pLNAβ with the restriction enzymes BlnI and BglII. The above two fragments cleaved were joined to a vector fragment prepared by treatment of pLNAβ with the restriction enzymes XbaI and BglII . A plasmid, pLNΔAβ, was thus constructed.

The lacZ gene prepared by cleaving pLNΔAβ with SpeI and XhoI was joined to pLenti6/TOPO-GFP at the SpeI-XhoI site. pLenti6/CMV-lacZ was thus constructed.

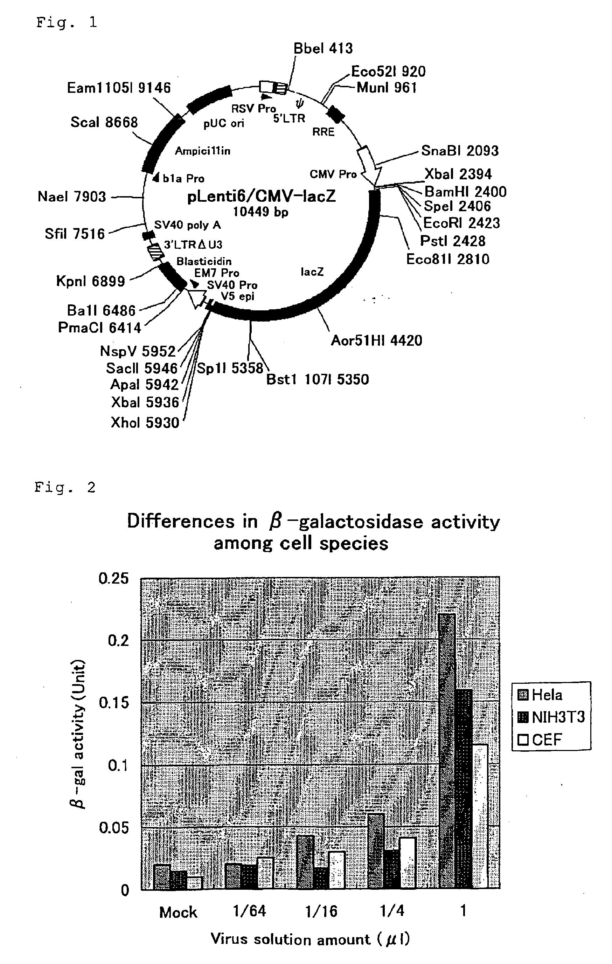

The structure of pLenti6/CMV-lacZ is shown in Fig. 1.

(Example 2) Preparation of replication-defective lentivirus vector for β-galactosidase expression

For preparing a lentivirus vector, 293FT packaging cells (product of Invitrogen Corporation) ($5 \times 10^6$ cells) were sown and cultured in culture dishes with a diameter of 100 mm. On the next day, at 90% confluence, the medium was exchanged for antibiotic-free fresh DMEM (Dulbecco's modified Eagle medium), and the defective vector constituent elements were introduced in the form of four plasmids (independently prepared from ViraPower Packaging Mix; 6.5 μg of gag/pol-encoding pLP1, 2.5 μg of Rev-encoding pLP2, 3.5 μg of pLP/VSV-G encoding coat protein VSV-G and 10 μg of pLenti6/CMV-lacZ prepared in Example 1, per culture dish) by the lipofection technique. On the day after gene transfer, medium exchange was made and, at 48 hours after transfection, the virus particle-containing culture supernatant was recovered and deprived of impurities by filtration through a 0.45-μm cellulose acetate filter (product of Advantec). This culture supernatant was centrifuged at 25,000 rpm at 4°C for 1.5 hours to cause precipitation of the virus. The supernatant was removed, 50 μl of TNE (50 mM Tris-HCl (pH 7.8), 130 mM NaCl, 1 mM EDTA) was added to the virus particle-containing precipitate and, after overnight standing at 4°C and after thorough suspending, the virus solution was recovered. Polybrene (product of Sigma) was added to the solution obtained to a concentration of 8 μg/mL, and the resulting mixture was used as the virus solution.

The thus-obtained high titer virus vector had a titer of $3.7 \times 10^6$ cfu/mL.

The virus titer measurement was carried out as follows. On the day before the measurement, HeLa cells (human-derived epithelial cell line, obtained from the American Type Culture Collection) were sown and cultured on 24-well dishes ($1.5 \times 10^4$ cells/dish). To each dish was added 1 mL of a $10^2$ to $10^6$-fold dilution of the virus solution and, after 48 hours, the cells were washed with two portions of PBS (phosphate-buffered physiological saline). Then, a fixing solution (200-fold dilution of 50% glutaraldehyde (product of Tokyo Kasei Kogyo Co., Ltd.) with PBS) was added in an amount sufficient for the cells to be immersed, followed by 10 minutes of standing at 4 °C. Thereafter, the fixing solution was discarded, the cells were washed with three portions of PBS, an X-Gal staining solution (5 mM of $K_3Fe(CN)_6$, 5 mM of $K_4Fe(CN)_6$, 2 mM of $MgCl_2$, 1 mg/mL of 5-bromo-4-chloro-3-indolyl-β-D-galactoside (product of Takara Shuzo Co., Ltd.) was then added and, after at least 3 hours of staining at 37°C, the proportion of the cells stained blue was determined. The titer was calculated as follows:

$$\text{Virus titer} = (\text{number of cells}) \times (\text{dilution factor}) \times (\text{expression ratio}) \; (\text{cfu/mL})$$

(Example 3) Preparation of chicken and quail fibroblasts

Chicken fibroblasts (CEF) and quail fibroblasts (QEF) were prepared as follows.

Fertilized chicken or quail eggs were incubated in an incubator (Showa Furanki model P-008) for 5 days (37.9°C, 65% humidity). Embryos were taken out of eggshells, transferred to culture dishes and washed with 0.1 M sterile phosphate buffer (pH 7.4), and the head, wings, limbs and internal organs were removed using scissors. Each embryo tissue was transferred to a centrifuge tube, 2 mL of a 0.25% trypsin solution (product of Sigma) was added, and cells were disrupted and suspended by repeating pipetting. The suspension was incubated at 37°C for 10 minutes, the reaction was then stopped by addition of Dulbecco's modified Eagle medium (product of Invitrogen Corporation), and cells were separated by 5 minutes of centrifugation at 1000 rpm. The supernatant was discarded, and Dulbecco's modified Eagle medium was again added, and the cells were sown on a culture dish. The cultivation was carried out in a 5% $CO_2$ atmosphere at 37°C.

(Example 4) Differences in lentivirus vector titer among cell species

The lentivirus vector prepared in Example 2 was introduced into cultured cells and the titer of the virus particles expressed was determined. In this way, the differences in the infectivity of the lentivirus vector among cell species were examined.

The cells used were HeLa (obtained from the American Type Culture Collection), NIH3T3 (NIHswiss mouse-derived fibroblastoid cell line, obtained from the American Type Culture Collection), and CEF and QEF cells prepared in Example 3, and $1.5 \times 10^4$ cells of each species were sown (Dulbecco's modified Eagle medium). On the next day, the medium was exchanged for a virus-containing medium and, after 2 days of incubation, the virus titer in the supernatant was assayed by the method described in Example 2.

In the same manner, each cell species was infected with the lentivirus for β-galactosidase expression constructed in Example 2, and the activity of the β-galactosidase expressed was measured and used as an indication of the infectivity of the lentivirus in each cell species.

The β-galactosidase activity measurement was carried out in the following manner.

Cultured cells were treated with a 0.25% trypsin solution (product of Sigma), and cells were collected by 10 minutes of centrifugation at 1500 rpm. Each pellet was washed with 0.1 M phosphate buffer (pH 7.5) and, after addition of 0.8 mL of a reaction buffer (10 mM of KCl, 1 mM of MgCl₂, 0.1% of Triton X-100 (product of Wako Pure Chemical Industries), 5 mM of 2-mercaptoethanol (product of Wako Pure Chemical Industries), 2 mM of phosphate buffer (pH 7.5)), cells were disrupted by sonication to give a cell solution.

A 0.6-mL portion of each cell solution was incubated at 37°C for 8 hours and, then, 0.1 mL of a preliminarily warmed solution of 4 mg/mL o-nitrophenyl β-D-galactopyranoside (ONPG) (product of Sigma) in 0.1 M phosphate buffer (pH 7.5) was added. After allowing the reaction to proceed, 0.3 mL of 1 M Na₂CO₃ (product of Wako Pure Chemical Industries) was added, and the absorption intensity at the wavelength of 420 nm was measured using an absorptiometer.

The β-galactosidase activity was expressed in terms of ONPG units (1 unit: activity corresponding to the formation of 1 μmol of o-nitrophenol per minute). Three runs were carried out, and the mean thereof was reported as the β-galactosidase activity.

Each cell species was infected with the lentivirus vector for β-galactosidase expression and the titer of the virus particles formed was determined. In this way, comparisons were made among the cell species with respect to the infectivity of the lentivirus.

The results are shown in Table 1. In Table 1, "Relative titer" refers to the percent titer in each cell species with the virus titer on the occasion of infection in HeLa being taken as 100.

Table 1

| | HeLa (Human) | NIH3T3 (Mouse) | CEF (Chicken) | QEF (Quail) |
|---|---|---|---|---|
| Virus titer ( cfu/mL ) | $8.5 \pm 2.1 \times 10^{5}$ | $3.5 \pm 0.5 \times 10^{4}$ | $2.5 \pm 0.5 \times 10^{5}$ | $2.2 \pm 0.5 \times 10^{4}$ |
| Relative titer(%) | 100 | 4.1 | 29.4 | 2.6 |

Examination of the relative infectivity levels in comparison with human-derived HeLa cells in which high infectivity of lentiviruses has been confirmed revealed that the lentivirus comprising a coat protein pseudotyped with VSV-G shows an infectivity of about 30% in chicken-derived fibroblasts as compared with the infectivity in HeLa.

Each cell species was infected with the lentivirus vector for β-galactosidase expression at various virus amount levels, and the enzyme activities due to the transgene were examined. Such enzyme activities serve as indicators of the infectivity of the lentivirus and of the gene transfer capacity. The results are shown in Fig. 2.

The VSV-G pseudotyped lentivirus vector caused the chicken fibroblasts to show a β-galactosidase activity about half that in HeLa.

Whereas there is no report about the infection of bird cells with lentivirus vectors, it was confirmed here that gene transfer can be accomplished with an infectivity of half to one third as compared with HeLa by pseudotyping the coat protein with VSV-G.

(Example 5) Preparation of vector construct pLenti/cDf-ΔAsPE-W for anti-prion scFvFc and green fluorescent protein (GFP) expression

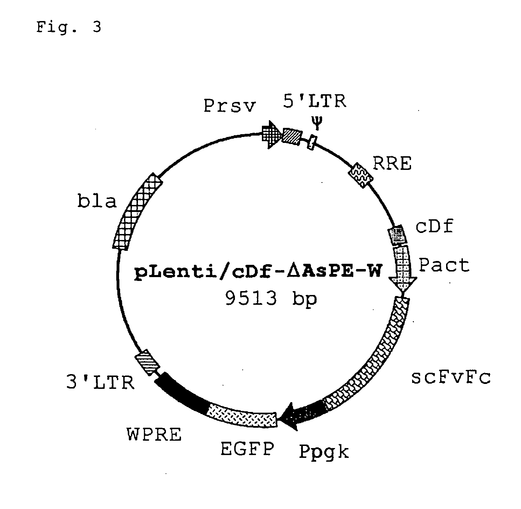

The vector construct pLenti/cDf-ΔAsPE-W for anti-prion scFvFc and GFP expression was constructed in the following manner. The scFvFc region of the construct pMSCV/GΔAscFv-Fc previously constructed by the present inventors (WO 2004/016081) was cleaved from that construct by cleavage with NheI and inserted into pBluescriptKS (-) (product of Stratagene) at the XbaI site to construct pBlue-scFvFc. The scFvFc region was cleaved from this pBlue-scFvFc by cleavage with XbaI and NotI, and the actin promoter region was cleaved from pMSCV/GΔAscFv-Fc with NotI and NheI. These two fragments were simultaneously inserted into pBluescriptKS (-) at the NotI site to construct pBlue-Pact-scFvFc. The GFP region of the above-mentioned pLenti6/TOPO-GFP was cleaved therefrom with SpeI and XbaI and inserted into pBluescriptKS(-) at the SpeI-XbaI site to construct pBlueGFP. The PGK promoter region was cleaved from pMSCVpuro (product of Clontech Laboratories, Inc.) with XhoI and PstI and inserted into pBlueGFP at the XhoI-PstI site to construct pBlue-Ppgk-GFP. The PGK promoter region and GFP were cleaved from this pBlue-Ppgk-GFP with XhoI and again inserted into pBluescriptKS(-) at the XhoI site to construct pBlueXPpg-GFP. The PGK promoter region and GFP were cleaved from pBlueXPpg-GFP with ClaI and KpnI and inserted into pLenti6/TOPO-GFP at the ClaI-KpnI site to construct pLenti-PpgkGFP. For replacing GFP with enhanced GFP (EGFP), EGFP was amplified by PCR using pIRES2-EGFP (product of Clontech Laboratories, Inc.) as a template as well as the primer EGFPdirect (BamHI) 5'-ATT<u>GGATCC</u>ACACGATGATAATATGGCCAC-3' (SEQ ID NO:9; the underlined portion being the BamHI site) and the primer EGFPreverse(KpnI) 5' -ATT<u>GGTACC</u>AGGCCGCTTTACTTGTACAG-3' (SEQ ID NO:10; the underlined portion being the KpnI site) . The amplified PCR product was cleaved with BamHI and KpnI and inserted into pLenti-PpgkGFP at the BamHI-KpnI site to construct pLenti-PpgkEGFP. The actin promoter and scFvFc regions were cleaved from pBlue-Pact-scFvFc with ClaI and inserted into pLenti-PpgkEGFP at the ClaI site to construct pLenti-PactscFvFc-PpgkEGFP. For insertion of a central DNA flap (cDf), the cDf was amplified by PCR using pLP1 (product of Invitrogen Corporation) as a template as well as the primer cDfdirect 5'-ACG<u>TCTAGA</u>AGACAGCAGTACAAAT-GGCAGTATT-3' (SEQ ID NO:11; the underlined portion being the XbaI site) and the primer cDfreverse 5'-AAGTCT<u>A-GA</u>CCAAACTGGATCTCTGCTGTCC-3' (SEQ ID NO:12; the underlined portion being the XbaI site). The amplified PCR product was cleaved with XbaI, and pLenti/cDf-AAsPE was constructed. Finally, the Woodchuck Hepatitis Virus Posttranscriptional regulatory element (WPRE) was amplified by PCR using the primer WPRE(direct KpnI) 5'-ACC<u>GGTACC</u>AATCAACCTCTGGATTACAAA-3' (SEQ ID NO:13; the underlined portion being the KpnI site) and the primer WPRE(reverse KpnI) 5'-ATA<u>GGTACC</u>CAGGCGGGGAGGC-3' (SEQ ID NO:14; the underlined portion being the KpnI site). This amplified product was cleaved with KpnI and inserted into pLenti/cDf-ΔAsPE at the KpnI site to construct pLenti/cDf-ΔAsPE-W (Fig. 3).

(Example 6) Construction of G0 transgenic chimeric chickens for the expression of scFvFc and GFP

First, the virus vector was prepared by the method described in Example 2. The titer measurement of the thus-prepared virus vector containing GFP as a marker was carried out in the following manner. On the day before the measurement, HeLa cells were sown and cultured on 24-well dishes ($1.5 \times 10^4$ cells/dish). To each dish was added 1 mL of a $10^2$ to $10^6$-fold dilution of the virus solution and, after 48 hours, the medium was exchanged for PBS, and the proportion of the cells manifesting the expression of the GFP gene was determined under a fluorescent microscope. The titer was calculated as follows:

```
Virus titer = (number of cells) x (dilution factor) x
              (expression ratio) (cfu/mL)
```

The titer of the thus-prepared virus was $2.2 \times 10^7$ cfu/ml to $7.7 \times 10^9$ cfu/ml.
This virus solution was introduced into chicken embryos just after laying by the method described in Japanese Kokai Publication 2002-176880. The chicken embryos after virus vector introduction were incubated for hatching by the method described in Japanese Kokai Publication 2002-176880. G0 transgenic chimeric chickens for scFvFc and GFP gene expression were thus constructed.

(Example 7) Serum scFvFc concentration measurement by ELISA

The G0 transgenic chimeric chickens constructed in Example 6 were raised for 20 to 40 days for growing chicks and, then, blood samples were obtained by blood drawing from the subalary vein. Each blood sample was incubated at 37°C for 1 hour and then centrifuges at 15,000 rpm for 5 minutes, and the supernatant was used as a serum sample.

Anti-Fc antibody (product of Cosmo Bio co., ltd.) diluted with PBS was placed in each well of an ELISA plate (100 µg/well), followed by overnight standing at 4°C. Each well was washed with three 200-µl portions of a 0.05% solution of Tween 20 in PBS and, then, 150 µl of PBS-0.05% Tween 20 solution-2% skim milk was added to each well. After 2 hours of standing at room temperature, each well was washed with three 200-µl portions of a 0.05% solution of Tween 20 in PBS, then 100 µl of each serum sample collected was added thereto, and the plate was allowed to stand at room temperature for 2 hours. Thereafter, each well was washed with three 200-µl portions of a 0.05% solution of Tween 20 in PBS, and 100 µl of peroxidase (POD)-labeled anti-human Fc antibody (product of Cosmo Bio co., ltd.) diluted with a 0.05% solution of Tween 20 in PBS was added to each well, and the plate was allowed to stand at room temperature for 1 hour. The wells were washed with four portions of a 0.05% solution of Tween 20 in PBS, and 100 µl of a color former solution (prepared by adding

10 µl of an aqueous solution of hydrogen peroxide (product of Wako Pure Chemical Industries) to a solution of 10 mg of o-phenylenediamine (product of KATAYAMA CHEMICAL INDUSTRIES Co., Ltd.) in 1 ml of methanol (diluted by distilled water to 100 ml)) was added to each well. After the lapse of 30 minutes to 1 hour, the reaction was stopped by adding 50 µl of 8 M concentrated sulfuric acid to each well, the fluorescence intensity at 490 nm was measured using a plate reader, and the concentration was calculated using a standard calibration curve. This standard calibration curve was constructed using standard Fc (product of Cosmo Bio co., ltd.).

The thus-measured serum scFvFc concentrations are shown in Table 2.

Table 2

| Individual's No. | Concentration (ng/ml) |
| --- | --- |
| B10 | 23.4 |
| B13 | 18.2 |
| B16 | 7.6 |
| B21 | 7.6 |
| B23 | 20.8 |
| B24 | 15.5 |
| B25 | 10.3 |
| B28 | 23.4 |
| B30 | 15.5 |
| B31 | 5 |

In this way, by carrying out gene transfer in fertilized chicken egg early embryos using a lentivirus vector comprising a coat protein pseudotyped with VSV-G, it becomes possible to construct G0 transgenic chimeric chickens capable of expressing the desired protein.

INDUSTRIAL APPLICABILITY

By incubating, for hatching, fertilized bird egg early embryos infected with a lentivirus vector comprising a coat protein pseudotyped with VSV-G according to the invention, it is possible to construct G0 transgenic chimeric birds.

Furthermore, the method of constructing G0 transgenic chimeric birds and offspring thereof makes it possible to construct birds capable of efficiently expressing a physiologically active protein, for example an antibody for medical use, in the blood and/or eggs as a result of introduction of the gene coding for such protein or antibody. Furthermore, the method of producing protein which uses the G0 transgenic chimeric birds and offspring thereof of the invention makes it possible to produce an antibody for medical use, for example, with good efficiency by constructing G0 transgenic chimeric birds and offspring thereof capable of producing the antibody and recovering and purifying the antibody from the serum and/or eggs of the birds.

**Claims**

1.  A lentivirus vector
    which comprises a coat protein containing VSV-G.

2.  The vector according to Claim 1
    which is replication-defective.

3.  The vector according to Claim 1 or 2
    which further contains a heterologous gene.

4.  The vector according to Claim 3
    wherein the heterologous gene contains a structural gene.

5.  The vector according to Claim 4
    wherein the heterologous gene further contains a marker gene.

6.  A recombinant bird host
    which is constructed by infecting a bird host with the vector according to any one of Claims 1 to 3.

7.  The recombinant bird host according to Claim 6
    wherein the vector contains a structural gene.

8.  The recombinant bird host according to Claim 6 or 7
    wherein the bird host is a fertilized egg early embryo.

9.  A transgenic chimeric bird
    which is constructed by infection with the vector according to any one of Claims 1 to 3, and
    offspring thereof.

10. The transgenic chimeric bird and offspring thereof according to Claim 9
    wherein the vector contains a structural gene.

11. A method of constructing a recombinant bird host
    which comprises infecting a bird host with the vector according to any one of Claims 1 to 4.

12. The method according to Claim 11
    wherein the bird host is a fertilized egg early embryo.

13. A method of constructing a G0 transgenic chimeric bird and offspring thereof
    which comprises infecting fertilized bird egg early embryos with a lentivirus vector comprising a coat protein containing VSV-G, and incubating the embryos.

14. The method of constructing a G0 transgenic chimeric bird and offspring thereof according to Claim 13
    wherein the lentivirus vector is replication-defective.

15. The method of constructing a G0 transgenic chimeric bird and offspring thereof according to Claim 13 or 14
    wherein the lentivirus vector is an HIV-derived one.

16. The method of constructing a G0 transgenic chimeric bird and offspring thereof according to any one of Claims 13 to 15
    wherein the lentivirus vector is one with the GFP gene and/or LacZ integrated therein.

17. The method of constructing a G0 transgenic chimeric bird and offspring thereof according to any one of Claims 13 to 16
    wherein the time for lentivirus vector infection is at a period succeeding the blastodermic stage.

18. The method of constructing a G0 transgenic chimeric bird and offspring thereof according to Claim 17
    wherein the site of lentivirus vector infection is in the heart or blood vessel formed in the early embryos.

**19.** The method of constructing a G0 transgenic chimeric bird and offspring thereof according to any one of Claims 13 to 18 wherein the method of lentivirus vector infection is the microinjection method.

**20.** The method of constructing a G0 transgenic chimeric bird and offspring thereof according to any one of Claims 13 to 19 wherein the bird is a chicken.

**21.** The method of constructing a G0 transgenic chimeric bird and offspring thereof according to any one of Claims 13 to 20 wherein a lentivirus vector having a titer of not lower than $1 \times 10^5$ cfu/mL is injected.

**22.** A G0 transgenic chimeric bird and offspring thereof
which is constructed by the method according to any one of Claims 13 to 21.

Fig. 1

Fig. 2

## Differences in β-galactosidase activity among cell species

Fig. 3

pLenti/cDf-ΔAsPE-W
9513 bp

Prsv  5'LTR
RRE
cDf
Pact
scFvFc
Ppgk
EGFP
WPRE
3'LTR
bla

## INTERNATIONAL SEARCH REPORT

| International application No. |
| --- |
| PCT/JP2004/012388 |

| A. CLASSIFICATION OF SUBJECT MATTER |
| --- |
| Int.Cl$^7$  C12N15/867, 5/10, A01K67/027 |

According to International Patent Classification (IPC) or to both national classification and IPC

| B. FIELDS SEARCHED |
| --- |
| Minimum documentation searched (classification system followed by classification symbols)<br>Int.Cl$^7$  C12N15/867, 5/10, A01K67/027 |
| Documentation searched other than minimum documentation to the extent that such documents are included in the fields searched |
| Electronic data base consulted during the international search (name of data base and, where practicable, search terms used)<br>MEDLINE(STN), BIOSIS(STN), WPIDS(STN), JICST FILE(JOIS) |

C. DOCUMENTS CONSIDERED TO BE RELEVANT

| Category* | Citation of document, with indication, where appropriate, of the relevant passages | Relevant to claim No. |
| --- | --- | --- |
| X<br>Y | MITTA B. et al., "Advanced modular self-inactivating lentiviral expression vectors for multigene interventions in mammalian cells and in vivo transduction.", Nucleic Acids Res., 2002, Vol.30, No.21, e113 | 1-8,11,12<br>9,10,13-22 |
| X<br>Y | COLEMAN JE., et al., "Analyses of the guanylate cyclase activating protein-1 gene promoter in the developing retina", Invest.Ophthalmol. Vis.Sci., 2002, Vol.43, No.5, p. 1335-43 | 1-8,11,12<br>9,10,13-22 |
| X | JP 2002-510199 A  (The University of North Carolinaat Chapel Hill),<br>02 April, 2002 (02.04.02),<br>Examples<br>& WO 98/51810 A1          & EP 981636 A<br>& US 2001/0044149 A1 | 1-5 |

☒ Further documents are listed in the continuation of Box C.          ☐ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>10 December, 2004 (10.12.04) | Date of mailing of the international search report<br>28 December, 2004 (28.12.04) |
| --- | --- |
| Name and mailing address of the ISA/<br>Japanese Patent Office | Authorized officer |
| Facsimile No. | Telephone No. |

Form PCT/ISA/210 (second sheet) (January 2004)

## INTERNATIONAL SEARCH REPORT

| | International application No. |
|---|---|
| | PCT/JP2004/012388 |

C (Continuation).  DOCUMENTS CONSIDERED TO BE RELEVANT

| Category* | Citation of document, with indication, where appropriate, of the relevant passages | Relevant to claim No. |
|---|---|---|
| X | WO 02/49677 A1 (RESEARCH DEVELOPMENT FOUNDATION),<br>27 June, 2002 (27.06.02),<br>Examples; Fig.<br>& WO 02/49677 A1          & US 2002/0114783 A1<br>& EP 1343532 A | 1-5 |
| Y | JP 2002-176880 A (Kaneka Corp.),<br>25 June, 2002 (25.06.02),<br>Examples<br>& WO 02/47475 A1          & AU 77669901 A | 9,10,13-22 |
| Y | MIZUARAI S., et al., "Production of transgenic quails with high frequency of germ-line transmission using VSV-G pseudotyped retroviral vector.", Biochem.Biophys.Res.Commun., 2001, Vol.286, No.3, p.456-63. | 9,10,13-22 |

Form PCT/ISA/210 (continuation of second sheet) (January 2004)

**INTERNATIONAL SEARCH REPORT**

| International application No. |
| --- |
| PCT/JP2004/012388 |

---

**Box No. II     Observations where certain claims were found unsearchable (Continuation of item 2 of first sheet)**

This international search report has not been established in respect of certain claims under Article 17(2)(a) for the following reasons:

1. ☐ Claims Nos.:
      because they relate to subject matter not required to be searched by this Authority, namely:

2. ☐ Claims Nos.:
      because they relate to parts of the international application that do not comply with the prescribed requirements to such an extent that no meaningful international search can be carried out, specifically:

3. ☐ Claims Nos.:
      because they are dependent claims and are not drafted in accordance with the second and third sentences of Rule 6.4(a).

---

**Box No. III     Observations where unity of invention is lacking (Continuation of item 3 of first sheet)**

This International Searching Authority found multiple inventions in this international application, as follows:
      The matter common to claims 1 to 22 resides in "a lentivirus vector containing VSV-G in the coat protein". As the results of the search, however, it is clarified that "a lentivirus vector containing VSV-G in the coat protein" is not novel because of already having been disclosed in document (JP 2002-510199 A (University of North Carolina at Chapel Hill) 02 April, 2002 (02.04.02)).
      Consequently, "a lentivirus vector containing VSV-G in the coat protein" falls within the category of prior art and, therefore, this common matter cannot be referred to as a special technical feature.
      Such being the case, the inventions as claimed in claims 1 to 22 are classified into two groups of inventions,     (continued to extra sheet)

1. ☒ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**       ☐   The additional search fees were accompanied by the applicant's protest.

                       ☒   No protest accompanied the payment of additional search fees.

---

Form PCT/ISA/210 (continuation of first sheet (2)) (January 2004)

# EP 1 672 076 A1

| INTERNATIONAL SEARCH REPORT | International application No. |
|---|---|
| | PCT/JP2004/012388 |

Continuation of Box No.III of continuation of first sheet(2)

i.e., a group of inventions relating to a lentivirus vector containing VSV-G in the coat protein as set forth in claims 1 to 5 and another group of inventions having a special technical feature of infecting a bird with a lentivirus vector containing VSV-G in the coat protein as claimed in claims 6 to 22.

SEQUENCE LISTING

<110> Kaneka Corporation

Nagoya Industrial Science Research Institute (Chubu Technology Licensing Office)

<120> METHOD OF CONSTRUCTING TRANSGENIC BIRD USING LENTIVIRUS VECTOR AND TRANSGENIC BIRD OBTAINED THEREBY

<130> B030335W001

<150> JP 2003-306573

<151> 2003-8-29

<160> 14

<210> 1

<211> 16

<212> DNA

<213> Artificial Sequence

<220>

<223> Designed sequence of a 5'-primer used for PCR amplification of the coding fragment of the green fluorescent protein

<400> 1

caccatgagc aagggc 16

<210> 2

<211> 20

<212> DNA

<213> Artificial Sequence


<220>

<223> Designed sequence of a 3'-primer used for PCR amplification of the coding fragment of the green fluorescent protein


<400> 2

tcacttgtac agctcgtcca 20


<210> 3

<211> 25

<212> DNA

<213> Artificial Sequence


<220>

<223> Designed sequence of a 5'-primer used for PCR amplification of the Miw promoter 5' region fragment


<400> 3

cggtctagag gaattcagtg gttcg 25


<210> 4

<211> 26

<212> DNA

<213> Artificial Sequence

<220>

<223> Designed sequence of a 3'-primer incorporating the BamH I recognition site at the 5' terminal used for PCR amplification of the Miw promoter 5' region fragment

<400> 4

ccaggatccg acgttgtaaa acgacg 26

<210> 5

<211> 28

<212> DNA

<213> Artificial Sequence

<220>

<223> Designed sequence of a 5'-primer incorporating the Hind III recognition site at the 5' terminal used for PCR amplification of the Miw promoter 3' region fragment

<400> 5

ccaaagcttg ccgcagccat tgcctttt 28

<210> 6

<211> 27

<212> DNA

<213> Artificial Sequence

<220>

<223> Designed sequence of a 3'-primer incorporating the Bln I recognition site at the 5' terminal used for PCR amplification of the Miw promoter 3' region fragment

<400> 6
atacctaggg gctggctgcg gaggaac 27

<210> 7
<211> 29
<212> DNA
<213> Artificial Sequence

<220>

<223> Designed sequence of a 5'-primer incorporating the Nhe I recognition site at the 5' terminal used for PCR amplification of the chicken β-actin promoter fragment lacking the intron

<400> 7
tttagctagc tgcagctcag tgcatgcac 29

<210> 8
<211> 27
<212> DNA
<213> Artificial Sequence

<220>

<223> Designed sequence of a 3'-primer incorporating the Xba I recognition site at the 5' terminal used for PCR amplification of the chicken β-actin promoter fragment lacking the intron

<400> 8

ataatctaga aacgcagcga ctcccgc 27

<210> 9

<211> 30

<212> DNA

<213> Artificial Sequence

<220>

<223> Designed sequence of a 5'-primer used for PCR amplification of the coding fragment of the enhanced green fluorescent protein

<400> 9

attggatcca cacgatgata atatggccac 30

<210> 10

<211> 29

<212> DNA

<213> Artificial Sequence

<220>

<223> Designed sequence of a 3'-primer used for PCR amplification of the coding fragm

ent of the enhanced green fluorescent protein

<400> 10

attggtacca ggccgcttta cttgtacag 29

<210> 11

<211> 34

<212> DNA

<213> Artificial Sequence

<220>

<223> Designed sequence of a 5'-primer used for PCR amplification of the coding fragm ent of the central DNA flap

<400> 11

acgtctagaa gacagcagta caaatggcag tatt 34

<210> 12

<211> 31

<212> DNA

<213> Artificial Sequence

<220>

<223> Designed sequence of a 3'-primer used for PCR amplification of the coding fragm ent of the central DNA flap

<400> 12

aagtctagac caaactggat ctctgctgtc c 31

<210> 13

<211> 30

<212> DNA

<213> Artificial Sequence

<220>

<223> Designed sequence of a 5'-primer used for PCR amplification of the coding fragment of the posttranscriptional regulatory element of woodchuck hepatites virus

<400> 13

accggtacca atcaacctct ggattacaaa 30

<210> 14

<211> 22

<212> DNA

<213> Artificial Sequence

<220>

<223> Designed sequence of a 3'-primer used for PCR amplification of the coding fragment of the posttranscriptional regulatory element of woodchuck hepatites virus

<400> 14

ataggtaccc aggcggggag gc 22